# EUROPEAN PATENT APPLICATION

(11) **EP 4 443 160 A1**
(43) Date of publication of application: **09.10.2024**
(21) Application number: 22901342.0
(22) Date of filing: 30.11.2022
(51) Int. Cl.: G01N 33/53, G01N 33/543

(54) **METHOD FOR MEASURING ELASTASE 1 IN FECES**

(30) Priority: 30.11.2021 JP 2021194361
(71) Applicant: PHC Corporation, Toon-shi Ehime 791-0395 (JP)
(72) Inventor: SATO, Atsushi, Toon-shi, Ehime 791-0395 (JP); NAGAHAMA, Yutaka, Toon-shi, Ehime 791-0395 (JP)
(74) Representative: Gille Hrabal Partnerschaftsgesellschaft mbB Patentanwälte
(86) International application number: PCT/JP2022/044078
(87) International publication number: WO 2023/100910

(57) **Abstract**

Provided are an immunoassay reagent and an immunoassay method for elastase 1 (E1) present in human fecal samples, which is simple and can handle a large number of samples without requiring special facilities or equipment, can analyze in a short time so as to be suitable for emergency testing, and can quantitatively analyze a wide range of concentrations from low to high. The immunoassay method, comprising:
(a) adding α1-antitrypsin (α1AT) to a fecal sample obtained from a subject;
(b) incubating the fecal sample and reacting E1 with α1AT to form a complex;
(c) incubating the fecal sample with a solution containing a carrier on which an immunological partner capable of recognizing the E1-α1AT complex is immobilized; and
(d) analyzing a change caused by the reaction between the E1-α1AT complex and the immunological partner.

## Description

### TECHNICAL FIELD

The present invention relates to a reagent for immunoanalysis and an immunoanalysis method of elastase 1 in feces and a method for detecting pancreatic diseases.

### BACKGROUND ART

Pancreatic diseases are known to be difficult to diagnose, even today, when various diagnostic methods have been developed. The reasons for this is that because the pancreas is located at the deepest part of the abdominal cavity, it is difficult to determine the presence or nature of disease using classical diagnostic methods such as palpation, visual inspection, auscultation, or the like, as well as X-ray examinations, or the like, that the clinical symptoms of pancreatic diseases are similar to those of other digestive system diseases, that there are no simple and reliable testing methods, or the like (Patent literature 1). In particular, the 5-year survival rate for pancreatic cancer is extremely short, and early detection is known to be essential to improving prognosis.

The pancreatic enzyme elastase 1 (CELA1; Chymotrypsin-like elastase family, member 1, hereafter sometimes and simply referred to as E1) is measured as a biomarker for diagnosing pancreatic diseases. E1 belongs to the serine protease family and is immunologically distinct from elastase, which is also present in leukocytes, platelets, the spleen, and the like. E1 is secreted from the pancreas into the duodenum in parallel with other digestive enzymes, but is leaked into the bloodstream due to pancreatic duct stenosis or pancreatitis. In the blood, most of it is bound to α1-antitrypsin (hereafter sometimes and simply referred to as α1AT), and measurement of its blood concentration is considered clinically useful. In particular, it is useful as an indicator for diagnosing or monitoring pancreatic diseases, as it frequently shows abnormally high values from a relatively early stage, reflecting pancreatitis associated with pancreatic cancer (especially the head of the pancreas) (Patent literature 2). However, because it is an invasive test, it is not widely practiced, and there are not many reports on blood E1 levels.

There is a linear correlation between E1 secretion and pancreatic lipase, amylase, and trypsin secretion, and furthermore, E1 secretion into the duodenum shows a correlation with the E1 concentration in feces (Patent literature 3). Exocrine pancreatic insufficiency (EPI) is associated with decreased secretion of E1, which is thought to result in the decreased enzyme concentration in the feces. Therefore, E1 has been reported to be used as a marker for diagnosing Exocrine pancreatic insufficiency (EPI) and for monitoring pancreatic exocrine function in patients with diabetes mellitus, cystic fibrosis, and chronic pancreatitis.

When E1 is used for diagnostic purposes in human fecal samples, it has been reported that its concentration is 5 to 6 times higher than that in pancreatic secretion. However, the details of this situation and its relationship to pancreatic diseases, such as whether the blood or fecal concentration is the most appropriate, are not understood enough at present.

The reasons why E1 in fecal samples has not been accurately measured and its actual status has not been understood include the influence of contaminants and the handling of fecal samples. Fecal samples usually contain water, food residues, intestinal mucosal cells, intestinal bacteria, and the like, but they also contain many solid impurities and components that are not absorbed into the body and are excreted. Furthermore, fecal samples have unique properties in that their components, shape, pH, and the like vary depending on the excretion conditions. Therefore, there are various influences and causes on the measurement results, and tests using fecal samples require different efforts than those for regular blood samples, such as suppressing nonspecific reactions or the like. Regarding the handling of fecal samples, for example, the dry extraction method of Kampanis et al. has been disclosed for the measurement of elastase 1 in human feces, but the actual handling is complicated and it is difficult to say that it is suitable for practical use when conducting large-scale sample testing (Non-Patent literature 1). In addition, in the case of the wet extraction method, wet or loose fecal samples must be dried, weighed, finally diluted with an extraction solution, and the like, which is very labor-intensive and may cause hygiene problems. Furthermore, it is difficult to standardize the application of standard concentrations between extraction methods, which is one of the reasons why accurate measurement is difficult.

When feces are used as samples for clinical testing, the effect of fecal occult blood must be considered. Fecal occult blood means that blood is present in the feces, but it may not be possible to measure it accurately due to the influence of intestinal bleeding, anal fissures, menstrual blood, or the like. In general, the positive rate of fecal occult blood tests is known to be about 5 to 10%. In addition to the possible effects of coexisting substances in the feces, the effects of substances in the blood of the subjects due to fecal occult blood must also be considered.

Under these circumstances, the development of a method and a reagent that can accurately measure E1 in human fecal samples have been awaited.

### CITATION LIST

### PATENT LITERATURE

[Patent literature 1] JP S63-73152 A
[Patent literature 2] WO 2002/079782
[Patent literature 3] JP 2017-516088 A

### NON-PATENT LITERATURE

[Non-patent literature 1] Ann. Clin. Biochem 46; 33-7, 2009

### SUMMARY OF INVENTION

### TECHNICAL PROBLEM

An object of the present invention is to provide an immunoassay reagent and an immunoassay method for E1 present in human fecal samples, which is simple and can handle a large number of samples without requiring special facilities or equipment, can analyze in a short time so as to be suitable for emergency testing, and can quantitatively analyze a wide range of concentrations from low to high.

### SOLUTION TO PROBLEM

The present inventors have conducted extensive study to solve the above problem. As a result, we found that it is possible to accurately measure E1 in human fecal samples in complex with E1 using α1AT, leading to the completion of the present invention.

The present invention relates to an immunoassay method in which E1 in human fecal samples is stabilized by forming a complex with its inhibitor, α1AT, and then analyzed by an antigen-antibody reaction using a monoclonal antibody specific to the E1-α1AT complex. Furthermore, the present invention relates to a method for detecting pancreatic diseases, which comprises analyzing E1 by the above-mentioned immunoassay method.

The present invention provides the following:
[1] A method for measuring pancreatic elastase 1 present in a fecal sample, comprising:
   (a) adding α1-antitrypsin to a fecal sample obtained from a subject;
   (b) incubating the fecal sample and reacting pancreatic elastase 1 with α1-antitrypsin to form a complex;
   (c) incubating the fecal sample with a solution containing a carrier on which an immunological partner capable of recognizing the pancreatic elastase 1-α1-antitrypsin complex is immobilized; and
   (d) analyzing a change caused by the reaction between the pancreatic elastase 1-α1-antitrypsin complex and the immunological partner.
[2] The method according to [1], wherein the step (a) is carried out in a pretreatment step for extracting the fecal sample.
[3] The method according to [1] or [2], comprising:
   diluting the fecal sample before adding α1-antitrypsin to the fecal sample in the step (a).
[4] The method according to any one of [1] to [3], wherein an amount of α1-antitrypsin added to the fecal sample in the step (a) is 20 µg or more.
[5] The method according to any one of [1] to [4], wherein an amount of α1-antitrypsin contained in the reaction solution to be subjected to the reaction step in the step (b) is 100 ng or more.
[6] A method for assisting in a detection of a pancreatic disease, comprising analyzing pancreatic elastase 1 by the method according to any one of [1] to [5].
[7] The method according to any one of [1] to [6], wherein the analysis carried out in the step (d) is any one of a chemiluminescent immunoassay, an electrochemiluminescent immunoassay, a fluorescent immunoassay, a radioimmunoassay, an immunochromatography, a Western blotting method, a latex agglutination method, and an immunoturbidimetric method.
[8] A reagent for immunological measurement, comprising a solid phase carrier on which an immunological partner capable of a pancreatic elastase 1-α1-antitrypsin complex is immobilized, and α1-antitrypsin.
[9] The reagent for immunological measurement according to [8], wherein an amount of α1-antitrypsin contained in a reaction solution to be subjected to a reaction step is 100 ng.
[10:] An extraction solution for pretreatment, used in a pretreatment step of a fecal sample subjected to a reagent for measurement of pancreatic elastase 1 or pancreatic elastase 1-α1-antitrypsin complex.
[11] The extraction solution for pretreatment according to [10], containing 41 to 975 mg of α1-anti trypsin.

### ADVANTAGEOUS EFFECTS OF INVENTION

The method of the present invention can easily assist in the detection of pancreatic diseases by measuring E1 present in human fecal samples. Accurate measurement of E1 in human fecal samples will enable its use as a marker for monitoring pancreatic exocrine function, which is expected to be useful in determining treatment plans and the like.

### BRIEF DESCRIPTION OF THE DRAWINGS

[Fig. 1] Figure 1 shows the measurement results of the E1 concentration against the concentrations of α1AT solutions added.
[Fig. 2] Figure 2 shows the recovery rate for each α1AT solution concentration to the theoretical value for each E1 sample.
[Fig. 3] Figure 3 shows a comparison of the measurement values when α1AT was added, using multiple measurement reagents.

### DESCRIPTION OF EMBODIMENTS

The present invention relates to an immunoassay method for measuring E1 present in human fecal samples in a complex formed with α1AT. Furthermore, the present relates to an immunological analytical reagent for measuring the E1-α1AT complex present in human fecal samples.

Hereinafter, the embodiments of the method for measuring E1 present in human fecal samples will be illustrated in detail, but the method is not limited these embodiments. For example, the present invention includes:
a method for detecting pancreatic diseases by measuring pancreatic elastase 1 present in fecal samples;
a method for assisting in detection of pancreatic diseases by measuring pancreatic elastase 1 present in fecal samples;
a method for measuring pancreatic elastase 1 present in fecal samples for the detection of pancreatic diseases;
an in vitro method for detecting pancreatic diseases by measuring pancreatic elastase 1 present in fecal samples;
a use of an immunological partner that recognizes a pancreatic elastase 1-α1-antitrypsin complex for the manufacture of a kit for the detection of pancreatic diseases; and
a method for measuring pancreatic elastase 1 present in fecal samples to provide information necessary for the detection of pancreatic diseases.

In the present specification, the term "measurement" (broad definition) includes, in addition to "measurement" (narrow definition) to quantitatively or semi-quantitatively determine the amount of an analyte, "detection" to judge the presence or absence of the analyte.

The samples used for the measurement of the present invention include human fecal samples, intestinal lavage fluid, and the like. For the fecal samples, there are no particular limitations on the specific form so long as it is derived from feces. For example, feces of any shape such as hardness (hard stool, normal stool, loose stool, diarrheal stool, watery stool, or the like), any water content, and the like can be used. The intestinal lavage fluid means a fluid that has passed through the intestinal lumen and has been collected. This includes oral intestinal lavage fluid that has passed through the intestinal lumen and has been collected. The intestinal lavage fluid may be collected from one excreted from the subject, or from the rectum of the subject on the verge of being excreted. In the present specification, when the term "fecal sample" is used to mean the "sample" used in the measurement (for example, in claim 1), it includes not only fecal samples but also intestinal lavage fluids and the like.

The fecal samples can be used by adopting common pretreatment procedures, such as extracting E1 or removing the insoluble fraction. The extraction solution used in the extraction procedure can be physiological saline, but buffers such as Good's buffer, phosphate, or the like, or solutions containing proteins such as BSA (Bovine Serum Albumin) or the like or surfactants can be used. Various conditions of the extraction solution, such as the concentration and pH of the buffer solution, and the concentration or the like of the surfactant or BSA can be appropriately set and used by those skilled in the art.

In the extraction procedure, the extraction solution is added to the fecal samples, and the fecal samples are sufficiently dispersed to elute E1 into the extraction solution. If necessary, a homogenizer or a vortex mixer may be used. In order to dissolve E1 more efficiently, the fecal samples may be allowed to stand for 30 minutes to an hour after being dispersed in the extraction solution.

Centrifugation or filter filtration can be used to remove the insoluble fraction. The centrifugation conditions, filter membranes used for filtration, and the like are not limited, as long as they can be used for pretreatment. For example, as the carriers constituting the filter, for example, polypropylene (PP), polyvinylidene fluoride (PVDF), glass fiber (GF), polyethersulfone (PES), nylon (NY), polytetrafluoroethylene (PTFE), regenerated cellulose (RC), cellulose acetate (CA), and methacrylate butadiene styrene (MBS) can be included. Furthermore, it may also be a hybrid type consisting of a combination of multiple of these components.

The above procedures can also be performed using a general stool collection kit. As an example of a stool collection kit, OC-Hemocatch (registered trademark) S (manufactured by Eiken Chemical Co., Ltd.), which is a stool collection kit for occult blood in feces, may be used. After pretreatment, the fecal samples should be preferably stored in a cool, dark place or frozen. More preferably, they should be stored in an ultra-low temperature freezer (-85 to -40°C). The frozen fecal samples can be thawed for use in the measurement.

The extraction solution used in these extraction procedures may be supplemented with α1AT as described below.

The method for measuring E1 of the present invention is not particularly limited, but it is possible to use an immunological partner capable of measuring E1. Immunological methods for detecting proteins include any method that uses labeled antibodies, such as enzyme-linked immunosorbent assay (ELISA), chemiluminescent immunoassay, electrochemiluminescent immunoassay, fluorescent immunoassay, radioimmunoassay, immunochromatography, and the like, as well as any known method that is commonly used, such as Western blotting, latex agglutination method, immunoturbidimetric method, and the like.

The term "immunological partner" as used in the present invention means a partner that immunologically and specifically binds to the substance to be measured, for example, an immunological substance (i.e., an antigen or antibody) that can specifically bind to various proteins, polysaccharides, lipids, nucleic acids, haptens, and complexes, fragments, or the like thereof. When the immunological partner is an antibody, the antibody used may be a monoclonal or polyclonal antibody, or a fragment thereof treated with an enzyme or the like. The antibody fragment is preferably a functional fragment containing the antigen-binding region of the antibody or its variable region, and examples thereof include F(ab')₂, Fab', Fab, and the like. F(ab')₂ and Fab' are produced by treating immunoglobulins with a proteolytic enzyme (for example, pepsin, papain, or the like), and are antibody fragments generated by digestion before and after the disulfide bond between two H chains in the hinge region. Furthermore, several immunological partners may be used in combination.

Hereinafter, we will explain the case where an antibody is used as the immunological partner as an example. In the method of the present invention, since the measurement is performed with the E1-α1AT complex as the substance to be measured, the antibody used may be any antibody capable of recognizing the E1-α1AT complex, and may be an anti-E1 antibody that specifically recognizes E1, or an anti-E1-α1AT complex antibody that specifically recognizes the complex of E1-α1AT. Any anti-E1 antibody can be used as long as it is not an antibody that does not recognize the E1-α1AT complex. The number of antibodies used may be only one type of antibody that specifically recognizes E1, or may be a combination of a first antibody that specifically recognizes E1 and a second antibody that recognizes E1 different from the first antibody. Furthermore, the antibody may be only one type that specifically recognizes the E1-α1AT complex, or it may be a combination of a first antibody that specifically recognizes the E1-α1AT complex and a second antibody that recognizes an E1-α1AT complex different from the first antibody. These may also be used in combination (hereinafter, these may be collectively referred to as "anti-E1 antibodies"). These are not particularly limited as long as the anti-E1 antibodies have different specific binding sites.

The anti-E1 antibodies can be produced, for example, by using as an immunogen a polypeptide containing part or all of the amino acid sequence of E1 or the E1-α1AT complex. The antigen polypeptide may be a synthetic polypeptide chemically synthesized according to a known method, or may be produced by genetic recombination or the like.

The antibody used in the present invention can be used as an immobilized antibody supported on an insoluble carrier such as a solid phase carrier or the like, or as a labeled antibody labeled with a labeling substance.

The immobilized antibody is an antibody that is supported on an insoluble carrier by physical adsorption, chemical bonding, or the like. These immobilized antibodies can be used to detect or quantify the substance to be measured in samples. Examples of insoluble carriers that can be used to support antibodies include polymeric materials such as latex, rubber, polyethylene, polypropylene, polystyrene, styrene-butadiene copolymers, polyvinyl chloride, polyvinyl acetate, polyacrylamide, polymethacrylate, styrene-methacrylate copolymers, polyglycidyl methacrylate, acrolein-ethylene glycol dimethacrylate copolymers, polyvinylidene difluoride (PVDF), silicone, and the like; agarose; gelatin; red blood cells; inorganic materials such as silica gel, glass, inert alumina, magnetic materials, and the like; and the like. One or more of these may be combined.

The method of the present invention can be carried out, for example, by supporting the anti-E1 antibody on latex particles. The latex particles that can be used in this case are not particularly limited, so long as they are latex particles that can be used in ordinary immunoassay reagents, and examples thereof include polystyrene, styrene-styrene sulfonate copolymers, and the like. The average particle size of the latex particles can be appropriately selected depending on the detection concentration of the substance to be measured or the measuring device. For example, particles with a particle size of 0.05 to 0.5 µm can be used. By using latex particles with different particle sizes, it is possible to accurately analyze values from low to high, which is preferable. In particular, on the high value side, it is preferable because it can prevent misinterpretation due to a decrease in agglutination ability even with a high concentration of elastase 1 (the so-called prozone phenomenon, in which an excessively high concentration reduces agglutination ability, resulting in an apparent decrease in agglutination and a measurement result that is lower than the actual value). The average particle size of latex particles in the present invention means a value measured by an electron microscope.

When the anti-E1 antibodies are immobilized on latex particles, for example, the latex particles may include two types of latex particles of different particle sizes carrying two types of anti-E1 antibodies having different specificities to E1 or the E1-α1AT complex, preferably may include at least (1) first latex particles carrying a first anti-E1 antibody to E1 or the E1-α1AT complex, and (2) second latex particles of a particle size different from that of the first latex particles carrying a second anti-E1 antibody to E1 or the E1-α1AT complex having a different specificity from that of the first anti-E1 antibody.

In the case of immunoassays using labeled antibodies, such as enzyme-linked immunosorbent assay (ELISA), chemiluminescent immunoassay, electrochemiluminescent immunoassay, fluorescent immunoassay, radioimmunoassay, immunochromatography, or the like, it is preferable to use a labeling substance. The labeling substance is not particularly limited as long as it is a labeling substance that can be used in ordinary immunological assays, and examples of such substances include enzymes, fluorescent substances, radioisotopes, insoluble granular substances, and the like. Examples of the labeling enzymes include alkaline phosphatase, peroxidase, glucose oxidase, tyrosinase, acid phosphatase, and the like. Examples of the fluorescent substances include fluorescein isothiocyanate (FITC), green fluorescent protein (GFP), luciferin, and the like. Examples of the radioisotopes include ¹²⁵I, ¹⁴C, ³²P, and the like.

When the labeling substance is an enzyme, the labeling substance can be measured by carrying out a luminescence, fluorescence, or color reaction using a substrate for the enzyme. For example, when the enzyme is alkaline phosphatase, the substrate may be a chemiluminescent substrate, such as CDP-star (Registered trademark) disodium (2-chloro-5-(4-methoxyspiro{1,2-dioxetane-3,2'-(5'-chloro)-tricyclo[3.3.1.13,7]decane}-4-yl)-1-phenylphosphate, CSPD (registered trademark) disodium (3-(4-methoxyspiro{1,2-dioxetane-3,2-(5'-chloro)tricyclo[3.3.1.13,7]decane}-4-yl)phenylphosphate, AMPPD (registered trademark) (adamantyl methoxyphenyl phosphoryl dioxycetane), APS-5, or the like; a fluorescent substrate, such as 4-methylumbelliferylphosphate or the like; a chromogenic substrate, such as p-nitrophenylphosphate, BCIP (5-bromo-4-chloro-3-indolyl-phosphate), NBT (4-nitroblue tetrazolium chloride), INT (iodonitrotetrazolium), or the like; can be used.

The immunological measurement method of the present invention can be carried out using an immunological measurement reagent consisting of one liquid or two or more liquids.

When the reagent for carrying out the present invention is composed of one liquid, it may be composed of, for example, a reaction reagent containing at least an insoluble carrier carrying an immunological partner for forming an immunological complex. According to a known method, the reagent is reacted with samples and a signal is detected.

Since it is difficult to directly and accurately measure E1 present in fecal samples, in the measurement of the present invention, E1 is measured in the form of a complex of E1 and α1AT.

The α1AT used in the present invention may be in any form that can be ultimately mixed with the sample, and may be contained in a pretreatment reagent used in the pretreatment step, may be contained in a stabilization reagent, or may be contained in the reagent containing the insoluble carrier described above. In the present invention, we found that in order to accurately measure E1 present in fecal samples, it is important to add a certain concentration range of α1AT to the sample or the reagent.

The α1AT used in the present invention may be added to the samples before the samples react with the reagent, or may be supplied in a state where it has been added to the reagent. It is sufficient that α1AT is contained in a reaction solution in which an immunological reaction with the E1-α1AT complex that is the substance to be measured is carried out. For example, α1AT may be added to any one of a diluent for pre-diluting the measurement samples, a reagent for mixing the measurement samples with an immobilized anti-E1 antibody bound to an insoluble carrier and reacting the E1-α1AT complex to be measured with the immobilized anti-E1 antibody, or a reagent for mixing and reacting a labeled antibody with the measurement samples and reacting the labeled antibody with the E1-α1AT complex to be measured; or to each of these reagents.

The α1AT used may be any α1AT that forms a complex with human pancreatic elastase 1, and preferably human-derived α1AT is used. Human α1AT may be α1AT present in blood purified from the blood, or may be recombinantly expressed using cultured cells or the like. Those skilled in the art can select and use the appropriate one.

The amount of α1AT added to form a complex with E1 is preferably sufficient to form a complex with E1. The lower limit of the amount to be added should be 20 µg or more, and the upper limit can be appropriately set depending on the amount of E1 contained in the samples. For example, the lower limit is preferably 41 µg or more, and more preferably 49 µg or more. The upper limit is preferably 975 µg or less. The lower and upper limits above may be combined as appropriate.

The lower limit of the amount of α1AT contained in the reaction solution may be appropriately set at a concentration at which there is a sufficient amount of α1AT to form a complex with E1 and the reactivity is confirmed compared to one when no α1AT is added, and should be 100 ng or more. For example, 514 ng or more is preferable, and 609 ng or more is more preferable. The upper limit of the amount of α1AT contained in the reaction solution is preferably 12188 ng or less, more preferably 6094 ng or less, and still more preferably 5143 ng or less. Those skilled in the art can determine the optimal amount of E1 to be added for each measurement reagent, taking into consideration the expected E1 concentration in fecal samples. The lower and upper limits above may be combined as appropriate.

Furthermore, the amount of α1AT to be added may be determined by the weight ratio of E1 and α1AT depending on the measurable range of the reagent used for measurement. In this case, for example, by adding α1AT in an amount 6 to 2400 times the weight of E1, it is possible to sufficiently form a complex between E1 and α1AT, and E1 in fecal samples can be accurately measured.

When the reagent of the present invention is composed of two or more liquids, it may be composed of, for example, a stabilizing reagent and a reaction reagent containing at least an insoluble carrier carrying an antibody or an antigen for forming an immunological complex. The stabilization reagent is used to dilute the samples to an appropriate concentration or to perform pretreatment, and can be prepared according to known methods. According to a known method, the sample is reacted with the stabilizing reagent, then with the reactive reagent, and a signal is detected. The α1AT used in the present invention may be added to the stabilizing reagent and/or the reaction reagent before the samples are reacted with the reaction reagent, and preferably, it can be supplied in a state where it has been added to the stabilizing reagent and/or the reaction reagent.

It is known that nearly 90% of E1 present in the blood forms a complex with α1AT, and measurement of the E1-α1AT complex is used in clinical testing as it is considered to be a measurement of E1. Serine protease inhibitors of the serpin superfamily, to which α1AT belongs, are covalently linked to the serine residue in the active center of the protease molecule, and upon loop insertion, are strongly attracted to the serpin molecule, destroying the structure near the active center. It is known that this causes dissociation of protease through hydrolysis. Thus, it is believed that E1 and α1AT are linked by a covalent bond and do not easily dissociate. Since it was thought that E1 and α1AT formed a complex even in fecal samples, it was unexpected that E1 could be measured by adding α1AT to fecal samples.

As a method for assisting the detection of pancreatic diseases using the E1 measurement method of the present invention, as the original data, statistically processed data, or the like for calculating a judgment threshold (cutoff value), the judgment threshold (cutoff value) calculated from statistical data or the like showing the correlation between E1 concentration in fecal samples and various diseases can be appropriately used. Those skilled in the art can appropriately set and use cutoff values based on the association with pancreatic diseases. For example, as a method for calculating these cutoff values is to create an ROC curve (Receiver Operating Characteristic Curve) from the E1 concentrations and perform analysis to set the cutoff value within the range where diagnostic sensitivity and specificity are effective.

Furthermore, it is also possible to use the measured E1 concentration as a value for risk assessment. It can be used as an indicator to determine whether pancreatic inflammation, pancreatic cancer, or the like is improving through medication or other treatments. For example, persistently high or increasing E1 concentrations suggest the need to reconsider treatment plans.

In addition to the anti-E1 antibody-carrying latex particles, the reagent of the present invention may further contain various additives that can be added to latex reagents, such as a buffer solution, an agglutination promoter (for example, a water-soluble polymer such as polyethylene glycol or the like), a non-specific reaction inhibitor (for example, an alkali metal salt, sugars, or the like), a protein [for example, bovine serum albumin (BSA)], or the like.

As the buffer solution, one having a buffering capacity of pH 6 to 8.5 is preferable. The buffer solution having a pH of 6 to 8.5 is a conventionally known buffer solution, such as a Tris buffer, phosphate buffer, Good's buffer, or the like.

In the reagent of the present invention, for example, when a Tris buffer is used, the Tris concentration in the Tris buffer is not particularly limited as long as it is a concentration that can achieve the specified Tris concentration described below in the system in which the latex agglutination reaction is carried out when the buffer is used, but is preferably 0.1 to 0.5 mol/L.

The concentration of Tris in the system in which the latex agglutination reaction is carried out is not particularly limited, so long as it is a concentration that can inhibit the autoagglutination reaction of latex particles, and can be appropriately selected depending on the concentrations of coexisting additives such as salts, proteins, sugars, and/or the like. The Tris concentration in the system in which the latex agglutination reaction is carried out is preferably 0.1 to 0.5 mol/L, and more preferably 0.2 to 0.3 mol/L. If the concentration is less than 0.1 mol/L, the latex particles may undergo self-agglutination. If the concentration exceeds 0.5 mol/L, the antigen-antibody reaction may be suppressed, resulting in poor detection sensitivity. The above-mentioned lower and upper limits of the Tris concentration can be appropriately combined, for example, 0.1 to 0.3 mol/L.

Furthermore, the pH of the buffer solution is preferably 6 to 8.5. If the pH is outside this range, the latex particles may self-aggregate, and problems may occur in terms of measurement accuracy.

When the reagent of the present invention contains a buffer solution of pH 6 to 8.5, the state of each anti-E1 antibody-carrying latex particle and the buffer solution of pH 6 to 8.5 in the reagent is not particularly limited, so long as each antibody-carrying latex particle, the buffer solution of pH 6 to 8.5, and the test sample can come into contact with each other during the latex agglutination reaction during use. That is, in this case, the form of the reagent of the present invention is not particularly limited, and may be, for example, a one-liquid reagent containing both each anti-E1 antibody-carrying latex particle and a buffer solution of pH 6 to 8.5, or may be a two-liquid reagent consisting of a first reagent containing each anti-E1 antibody-carrying latex particle and a second reagent which is a buffer solution of pH 6 to 8.5.

In the measurement method of the present invention, the test samples are contacted with each of the latex particles carrying the antibody, preferably under conditions of pH 6 to 8.5, to cause an antigen-antibody reaction and the resulting latex agglutination reaction, and E1 in the test samples can be analyzed by analyzing the degree of agglutination.

In the measurement method of the present invention, when the latex agglutination reaction is carried out under conditions of pH 6 to 8.5 using a buffer solution of pH 6 to 8.5, the order of contacting each anti-E1 antibody-carrying latex particle with the buffer solution of pH 6 to 8.5 and the test samples is not particularly limited, as long as the antigen-antibody reaction does not proceed in the absence of the buffer solution of pH 6 to 8.5 (i.e., the anti-E1 antibody-carrying latex particle is not contacted with the test sample first). For example, each anti-E1 antibody-carrying latex particle can be contacted in advance with a buffer solution of pH 6 to 8.5, and the mixture can then be contacted with the test samples, or the test samples can be contacted in advance with a buffer solution of pH 6 to 8.5, and the mixture can then be contacted with each anti-E1 antibody-carrying latex particle.

The conditions for the antigen-antibody reaction in the measurement method of the present invention can be the same as those for the implementation of a conventional immunological latex turbidimetric method. For example, the reaction is preferably carried out at a pH of 6 to 8.5. The reaction temperature is preferably 0 to 50°C, and more preferably 20 to 40°C. The reaction time can be appropriately determined, for example, the measurement can be completed in 10 to 15 minutes using a general-purpose automatic analyzer. The above-mentioned lower and upper limits of the reaction temperature can be appropriately combined, for example, 0 to 40°C.

The degree of agglutination caused by the antigen-antibody reaction can be analyzed by known analytical methods, such as optical analysis. The optical analysis method can be, for example, a method in which a reaction solution is irradiated with light and the scattered light or transmitted light is analyzed. More specifically, the analysis can be performed using an optical instrument that measures the scattered light intensity, absorbance, or transmitted light intensity. The preferred measurement wavelength is 300 to 800 nm. The analysis using the optical instrument can be carried out by measuring the increase or decrease in scattered light intensity, absorbance, or transmitted light intensity by selecting the size and/or concentration of the latex particles used and setting the reaction time according to a known method. It is also possible to use these methods in combination.

In the method for detecting pancreatic diseases according to the present invention, serum or plasma is used as test samples, and pancreatic diseases (particularly acute pancreatitis) can be detected (diagnosed) by analyzing E1 in fecal samples by the measurement method of the present invention.

Based on these findings, rapid and accurate measurement of E1 in human fecal samples using the present invention will not only aid in the diagnosis of pancreatic diseases, but will also provide more reliable measurement values in monitoring the progress of treatment, making it possible to provide extremely useful information.

### EXAMPLES

The present invention will now be further illustrated by, but is by no means limited to, the following Examples.

### [Example 1: Experiment of adding α1AT to fecal extraction solution]

Iatro IRE1II (hereinafter referred to as IRE1, manufactured by LSI Medience Corporation) was used as a reagent for measuring E1 in fecal samples, and it was confirmed whether measurement values of Iatro IRE1II were obtained by adding α1-antitrypsin to a fecal extraction solution.

A purified product of human plasma-derived α1AT (manufactured by Sigma-Aldrich) was dissolved in a Tris buffer to prepare α1AT solutions with concentrations of 0, 16, 130, 325, 813, 3250, and 6500 µg/mL calculated based on the absorption coefficient of α1AT. To 150 µL of human fecal extract solutions (hereafter referred to as fecal extract solutions), 150µL of the prepared α1AT solutions of each concentration were added to prepare α1AT-added fecal extract solutions. To bring the α1AT-added fecal extract solutions into the measurement range of the IRE1 reagent, the solutions were further diluted 80-fold. A commercially available D-D dimer diluent (manufactured by LSI Medience corporation) was used for dilution. The α1AT-added fecal extract solutions diluted 80-fold were measured at a multiplicity of 2 using the IRE1 reagent installed in a Hitachi 7180 automatic analyzer (hereafter referred to as H7180, manufactured by Hitachi High-Tech Corporation).

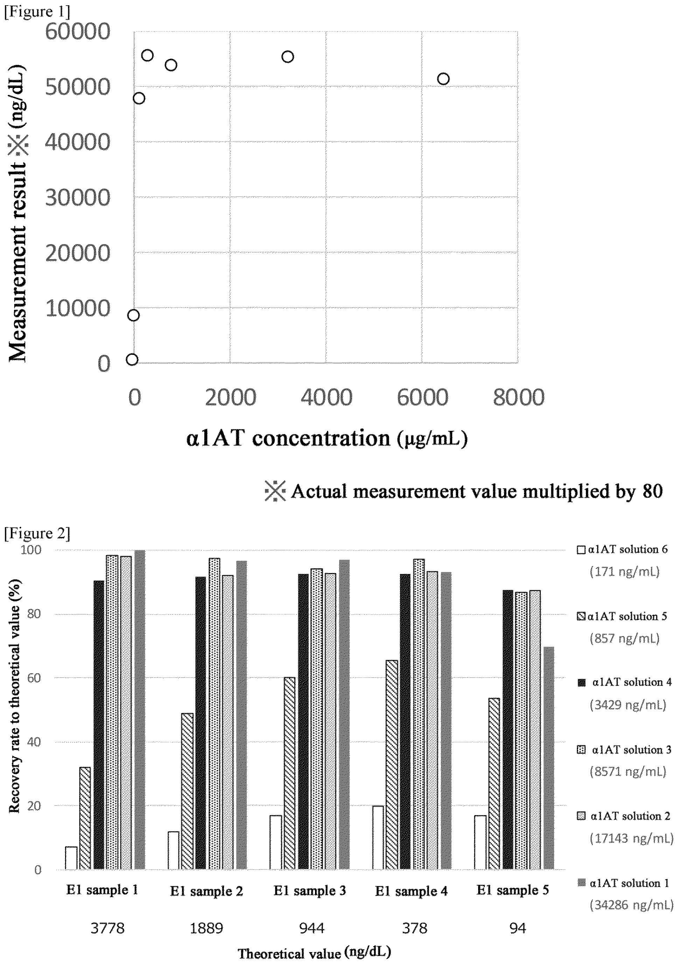

Figure 1 shows the measurement results of the IRE1 reagent against the concentrations of α1AT solutions added to the fecal extraction solution. It was confirmed that the addition of α1AT to the fecal extract solution increased the measurement values of the IRE1 reagent, and the maximum was observed when the concentrations of α1AT solutions was 325-3250 µg/mL. At the maximum concentration of 6500 µg/mL among the experimental conditions, the measurement value was 7.5% lower than that at 3250 µg/mL, but it was confirmed that the recovery rate was sufficient for use in E1 measurement. The amounts of α1AT added to the samples used in the measurement process were 49, 122, 488, and 975 µg, but because they were diluted 80-fold for measurement, the actual amounts were 609, 1523, 6094, and 12188 ng. This confirms that the addition of α1AT can provide the measurement values of IRE1 reagent in fecal extraction samples.

### [Example 2: Study of α1AT solution concentration]

The optimal concentration of α1AT to be added to the IRE1 reagent in the measurement range of 80 to 4000 ng/dL was examined to determine the concentration of α1AT solution to be used in future experiments.

Elastase 1 purified from human pancreatic secretion was used to prepare E1 samples with concentrations of 14, 57, 142, 285, and 571 ng/mL calculated based on the absorption coefficient of E1 using a phosphate buffer. Similarly, α1AT solutions were prepared using a phosphate buffer with concentrations of 171, 875, 3429, 8571, 17143, and 34268 ng/mL calculated based on the absorption coefficient of α1AT. To 150 µL of each E1 sample, 150 µL of each α1AT solution was added to prepare E1-α1AT mixed solutions.

The H7180 was equipped with the IRE1 reagent, and the E1-α1AT mixed solutions were measured at a multiplicity of 3. The measurement value of the mixed solution of 571 ng/mL E1 sample 1 and 34268 ng/mL α1AT solution 1 was taken as the theoretical value of E1 sample 1 (3778 ng/dL). The theoretical value of each E1 sample was set based on the dilution ratio from E1 sample 1.

Figure 2 shows the for each E1 sample. For E1 samples 1 to 4 with theoretical values of 378 to 3778 ng/dL, α1AT solutions 1 to 4 with α1AT concentrations of 3429 to 34286 ng/mL showed recovery rates of 90% or more. In E1 sample 5 with a theoretical value of 94 ng/dL, the maximum recovery was 87.6% for α1AT solutions 2 to 4 with α1AT concentrations of 3429 to 17143 ng/mL, and 69.7% for 34286 ng/mL α1AT solution, which were confirmed to be sufficient recovery rates for E1 measurement. When the E1 concentration is in the measurement range of IRE1 reagent, which is 80 to 4000 ng/dL, the α1AT concentration of 3429 to 17143 ng/mL is a good recovery rate against the theoretical value. Specifically, the amounts of α1AT added when the recovery rate of E1 was 70% or more were 514, 1286, 2571, and 5143 ng, respectively. Therefore, we tentatively used 8600 ng/mL as the concentration of α1AT solution in the subsequent experiments, so that 1290 ng was included in samples used for measurement.

### [Example 3: Comparative experiment between IRE1 reagent and fPELA reagent]

The reactivity of the evaluation system that adds α1AT using the IRE1 reagent was compared with that of an existing fecal elastase 1 LTX reagent.

fPELA turbo (hereinafter referred to as fPELA, manufactured by BUBLMANN) was used as the existing fecal E1 measurement reagent.

E1 purified from human pancreatic secretion was used to prepare E1 samples with concentrations of 14, 57, 142, 285, and 571 ng/mL calculated based on the absorption coefficient of E1 using a phosphate buffer. Similarly, α1AT solutions were prepared using a phosphate buffer with a concentration of 8600 ng/mL calculated based on the absorption coefficient of α1AT. To 200 µL of each E1 solution, 200 µL of the α1AT solution was added to prepare E1-α1AT mixed solutions.

Using the fPELA reagent installed in an automatic analyzer cobas c501 (hereinafter referred to as c501, manufactured by Roche), each E1 sample, the E1-α1AT mixed solutions, a standard for IRE1 reagent, and a control for IRE1 reagent were measured at a multiplicity of 2.

Using the IRE1 reagent installed in H7180, the E1-α1AT mixed solutions were measured at a multiplicity of 2.

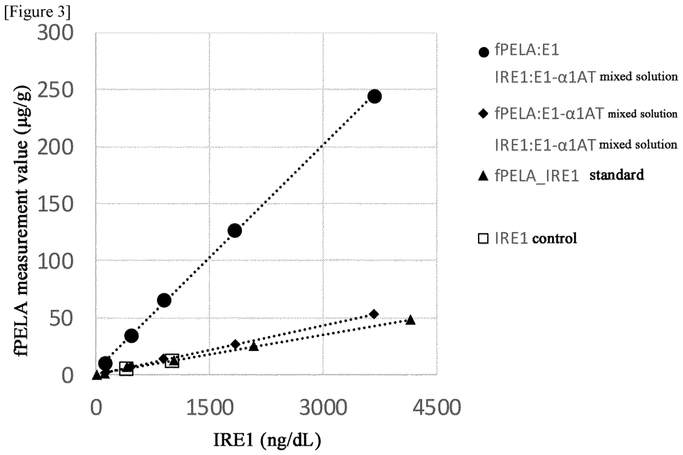

In Figure 3, against the value of IRE1, the measure values of each E1 sample, the E1-α1AT mixed solutions, the standard for IRE1 reagent, and the control for IRE1 reagent were plotted. For the measurement values of E1-α1AT mixed solutions in the IRE1 reagent, and the measurement values of E1 samples by the fPELA reagent, good linearity was obtained in a concentration-dependent manner. When the fPELA reagent was used to measure the E1-α1AT mixed solutions, the measurement values were lower when the E1 solutions were measured directly. The measurement values of the E1-α1AT mixed solutions by the fPELA reagent were close to those of the standard and control for IRE1 reagent. In the standard and control for IRE1 reagent, E1 was contained as a complex with α1AT, and the fPELA reagent was found to be affected by α1AT. In the case of fecal samples, there may be cases where fecal occult blood is present in the sample, and the measurement value may be affected by the presence of α1AT in the blood, but it was confirmed that by using the reagent that recognizes the E1-α1AT complex, the measurement values were not affected even in samples containing fecal occult blood.

The measurement values of the IRE1 reagent, which recognizes the E1-α1AT complex, correlated with the measurement values of the fPELA reagent, which recognizes and measures E1, confirming that the results obtained by adding α1AT to fecal samples and measuring them are reliable data.

### INDUSTRIAL APPLICABILITY

By using the measurement reagent and the measurement method of the present invention, it is possible to measure the concentration of E1 in fecal samples without being affected by measurement-interfering substances, and the reagent and method can be used to assist in the diagnosis of pancreatic diseases.

Furthermore, the ability to accurately measure E1 concentrations in fecal samples from healthy persons can provide highly reliable measurement values that can be used not only to diagnose pancreatic diseases, but also to select appropriate treatments, monitor the effectiveness of treatment, and predict prognosis, providing extremely useful information for medical treatment.

## Claims

1. A method for measuring pancreatic elastase 1 present in a fecal sample, comprising:
(a) adding α1-antitrypsin to a fecal sample obtained from a subject;
(b) incubating the fecal sample and reacting pancreatic elastase 1 with α1-antitrypsin to form a complex;
(c) incubating the fecal sample with a solution containing a carrier on which an immunological partner capable of recognizing the pancreatic elastase 1-α1-antitrypsin complex is immobilized; and
(d) analyzing a change caused by the reaction between the pancreatic elastase 1-α1-antitrypsin complex and the immunological partner.

2. The method according to claim 1, wherein the step (a) is carried out in a pretreatment step for extracting the fecal sample.

3. The method according to claim 1 or 2, comprising:
diluting the fecal sample before adding α1-antitrypsin to the fecal sample in the step (a).

4. The method according to any one of claims 1 to 3, wherein an amount of α1-antitrypsin added to the fecal sample in the step (a) is 20 µg or more.

5. The method according to any one of claims 1 to 4, wherein an amount of α1-antitrypsin contained in the reaction solution to be subjected to the reaction step in the step (b) is 100 ng or more.

6. A method for assisting in a detection of a pancreatic disease, comprising analyzing pancreatic elastase 1 by the method according to any one of claims 1 to 5.

7. The method according to any one of claims 1 to 6, wherein the analysis carried out in the step (d) is any one of a chemiluminescent immunoassay, an electrochemiluminescent immunoassay, a fluorescent immunoassay, a radioimmunoassay, an immunochromatography, a Western blotting method, a latex agglutination method, and an immunoturbidimetric method.

8. A reagent for immunological measurement, comprising a solid phase carrier on which an immunological partner capable of a pancreatic elastase 1-α1-antitrypsin complex is immobilized, and α1-antitrypsin.

9. The reagent for immunological measurement according to claim 8, wherein an amount of α1-antitrypsin contained in a reaction solution to be subjected to a reaction step is 100 ng.

10. An extraction solution for pretreatment, used in a pretreatment step of a fecal sample subjected to a reagent for measurement of pancreatic elastase 1 or pancreatic elastase 1-α1-antitrypsin complex.

11. The extraction solution for pretreatment according to claim 10, containing 41 to 975 mg of α1-antitrypsin.
